# EUROPÄISCHE PATENTANMELDUNG

(11) **EP 2 295 083 A1**
(43) Veröffentlichungstag der Anmeldung: **16.03.2011**
(21) Anmeldenummer: 09170260.5
(22) Anmeldetag: 15.09.2009
(51) Int. Cl.: A61K 45/06

(54) **Pharmazeutische Zusammensetzung mit den Wirkstoffen Metformin und Sitagliptin oder Vildagliptin**

(71) Anmelder: Ratiopharm GmbH, 89079 Ulm (DE)
(72) Erfinder: Rimkus, Katrin, 80798, München (DE); Stefan, Ralf, 88370, Ebenweiler (DE)
(74) Vertreter: Teipel, Stephan

(57) **Zusammenfassung**

Die vorliegende Erfindung betrifft eine pharmazeutische Zusammensetzung, die den Wirkstoff Metformin in Kombination mit einem der Wirkstoffe Sitagliptin oder Vildagliptin umfasst. Darüber hinaus betrifft die vorliegende Erfindung ein Verfahren zur Herstellung der genannten pharmazeutischen Zusammensetzung.

## Beschreibung

Die vorliegende Erfindung betrifft eine pharmazeutische Zusammensetzung, die den Wirkstoff Metformin in Kombination mit einem der Wirkstoffe Sitagliptin oder Vildagliptin umfasst. Darüber hinaus betrifft die vorliegende Erfindung ein Verfahren zur Herstellung der genannten pharmazeutischen Zusammensetzung.

Metformin ist ein Arzneistoff aus der Gruppe der Biguanide, der bei nicht insulinabhängiger Zuckerkrankheit (Diabetes mellitus Typ 2) und insbesondere bei Übergewicht und Fettsucht eingesetzt wird. Metformin ist einer der am längsten eingesetzten Antidiabetika. Bei Metformin handelt es sicht um das 1,1-Dimethylbiguanid folgender Strukturformel:

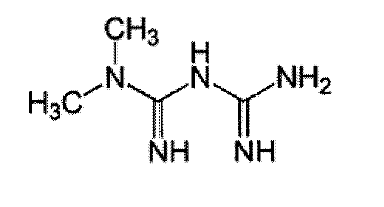

Metformin steht in Wirkstärken von 500 mg, 850 mg und 1000 mg zur Verfügung, um eine individuelle Blutzuckereinstellung vornehmen zu können. Die Tabletten werden oral verabreicht. Metformin wird zunächst als Monosubstanz eingesetzt. Sollte sich damit keine ausreichende Blutzuckersenkung einstellen, so ist bekannt, den Wirkstoff mit anderen oralen Antidiabetika, wie den Dipeptidyl-peptidase-4-Inhibitoren zu kombinieren.

Bekannte Dipeptidyl-peptidase-4-Inhibitoren sind beispielsweise Sitagliptin und Vildagliptin. Bei Sitagliptin handelt es sich um (R)-3-Amino-1-[3-(trifluormethyl)-5,6,7,8-tetrahydro[1,2,4]triazol[4,3-a]pyrazin-7-yl]-4-(2,3,5-trifluorphenyl)butan-1-on, das folgende Strukturformel aufweist:

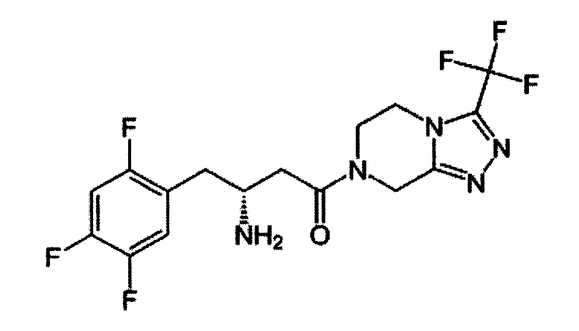

Bei Vildagliptin handelt es sich um (2S)-{((3-Hydroxyadamantan-1-yl)amino)acetyl}pyrrolidin-2-carbonitril mit der folgenden Strukturformel:

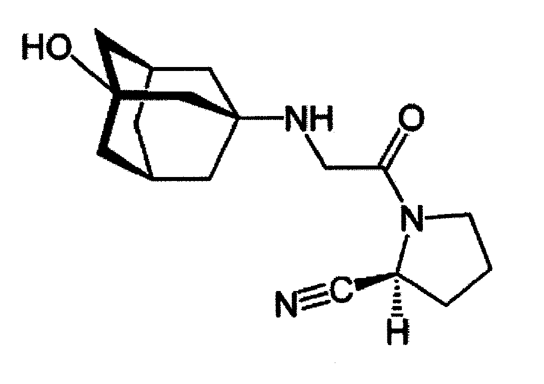

Sitagliptin ist unter den Handelsbezeichnungen Januvia^{®} und Xelevia^{®} erhältlich. Kombinationspräparate aus Sitagliptin und Metformin sind unter den Handelsbezeichnungen Janumet^{®} und Velmecia^{®} erhältlich. Vildagliptin ist als Arzneimittel unter der Handelsbezeichnung Galvus^{®} erhältlich, ein Kombinationspräparat aus Vildagliptin und Metformin ist unter der Handelsbezeichnung Eucreas^{®} erhältlich.

Kombinationspräparate aus Metformin und Vildagliptin sind in der WO 2007/041053 beschrieben. Die offenbarten Tabletten können neben den Wirkstoffen übliche Hilfsstoffe, wie beispielsweise Füllmittel, Bindemittel, Sprengmittel, Schmiermittel und Farbstoffe enthalten. Als Schmiermittel werden beispielhaft kolloidales Siliziumdioxid, Magnesiumtrisilicat, Stärke, Talkum, Calciumphosphat, Magnesiumstearat, Aluminiumstearat, Calciumstearat, Magnesiumcarbonat, Magnesiumoxid, Polyethylenglycol, Cellulose und mikrokristalline Cellulose genannt. Das Schmiermittel soll in einer Menge von bis zu 6 Gew.-% vorhanden sein können. In den Beispielen werden die Tabletten durch Nassgranulationsverfahren hergestellt.

Die WO 2007/078726 offenbart Kombinationspräparate, die 3 bis 20 Gew.-% Dipeptidyl-peptidase-4-Inhibitor, 25 bis 94 Gew.-% Metforminhydrochlorid, 0,1 bis 10 Gew.-% Schmiermittel und 0 bis 35 Gew.-% Bindemittel enthalten. Als Schmiermittel sind Magnesiumstearate, Calciumstearate, Stearinsäure, Natriumstearylfumarat und hydriertes Rizinusöl genannt. Bevorzugt enthalten die Tabletten, die in den Beispielen durch Nassgranulationsverfahren hergestellt werden, nur bis zu 2 Gew.-% Schmiermittel.

Die im Stand der Technik beschriebenen Kombinationspräparate weisen den Nachteil auf, dass die Tabletten nur durch Nassgranulation hergestellt werden können, da es sich bei dem Wirkstoff Metformin um einen sehr schlecht verpressbaren Wirkstoff handelt. Bei der Nassgranulation besteht jedoch die Gefahr, dass sich der Wirkstoff entweder durch Wechselwirkungen mit dem eingesetzten Lösungsmittel zersetzt und unerwünschte Abbauprodukte gebildet werden.

Es besteht somit weiterhin ein Bedürfnis nach pharmazeutischen Zusammensetzungen, die den Wirkstoff Metformin in Kombination mit einem der Wirkstoffe Sitagliptin oder Vildagliptin enthalten, und die durch ein einfaches Verfahren, vorzugsweise durch Direktverpressung, hergestellt werden können. Außerdem ist es wünschenswert, dass die erhaltenen Tabletten eine zum Lackieren geeignete Härte von > 100 kN aufweisen. Gleichzeitig dürfen die Tabletten durch hinzufügen weiterer Hilfsstoffe nicht so groß werden, dass sie beim Verabreichen Schluckbeschwerden verursachen. Schließlich müssen die Hilfsstoffe so gewählt werden, dass eine schnelle Freisetzung der Wirkstoffe aus der Tablette gewährleistet ist.

Diese Aufgaben wurden erfindungsgemäß überraschend dadurch gelöst, dass die Wirkstoffe zusammen mit mehr als 10 Gew.-% Schmiermittel zu der pharmazeutischen Zusammensetzung verarbeitet werden, wobei das Schmiermittel Polyethylenglykol oder eine Mischung aus Polyethylenglykol mit einem oder mehreren anderen Schmiermitteln ist. Der Einsatz hoher Schmiermittelkonzentrationen in der pharmazeutischen Zusammensetzung ist umso überraschender, als bekannt ist, dass dem Vorteil ihrer Schmierwirkung häufig der Nachteil der Hydrophobierung des Gutes und damit einer Verlängerung der Zerfallszeit bzw. der Auslösegeschwindigkeit der Tablette gegenübersteht, weshalb Schmiermittel in möglichst geringer Konzentration eingesetzt werden sollen (Schmidt Christin, Wirk- und Hilfsstoffe, Wissenschaftliche Verlagsgesellschaft mbH, Stuttgart, 1999). Entgegen dieser bekannten Nachteile von Schmiermitteln wurde vorliegend gefunden, dass, wenn das Schmiermittel Polyethylenglykol ist oder umfasst, dieses in hoher Konzentration von mehr als 10 Gew.-% eingesetzt werden kann und dabei trotzdem Tabletten mit hoher Auflösegeschwindigkeit erhalten werden, die sich zudem durch Direktverpressen herstellen lassen und die eine beispielsweise für die Lackierung vorteilhafte Härte aufweisen

Die vorliegende Erfindung betrifft somit eine pharmazeutische Zusammensetzung, die den Wirkstoff Metformin oder ein pharmazeutisch verträgliches Salz davon in Kombination mit einem der Wirkstoffe Sitagliptin oder Vildagliptin oder einem pharmazeutisch verträglichen Salz eines dieser Wirkstoff sowie mehr als 10 Gew.-% Schmiermittel bezogen auf das Gesamtgewicht der Zusammensetzung umfasst, wobei das Schmiermittel Polyethylenglykol oder eine Mischung aus Polyethylenglykol mit einem oder mehreren anderen Schmiermitteln ist.

Bei der erfindungsgemäßen pharmazeutischen Zusammensetzung handelt es sich um eine Zusammensetzung mit einer festen Dosis der Wirkstoffe, wobei beide Wirkstoffe gemeinsam in einer Dosiseinheit, insbesondere einer Tablette enthalten sind.

Der Wirkstoff Metformin wird in der erfindungsgemäßen pharmazeutischen Zusammensetzung vorzugsweise als pharmazeutisch verträgliches Salz und insbesondere als Hydrochloridsalz eingesetzt.

Der Wirkstoff Sitagliptin wird vorzugsweise in Form eines seiner pharmazeutisch verträglichen Salze eingesetzt. Pharmazeutisch verträgliche Salze des Sitagliptins sind beispielsweise in WO 2003/004498 beschrieben. Besonders bevorzugt wird Sitagliptin als sein Phosphatsalz, insbesondere als Phosphatmonohydrat eingesetzt. Das entsprechende Salz sowie seine Herstellung sind in der WO 2005/003135 offenbart. Alternativ kann der Wirkstoff Sitagliptin als Hydrochlorid, Sulfat, Mesylat, Besylat, Tosylat oder Mono-, Di- oder Tricarbonsäuresalz eingesetzt werden. Geeignete Carbonsäuren haben die Struktur R¹-COOH, worin R¹ Wasserstoff, Carboxyl, C₁₋₄-Alkyl oder C₂₋₄-Alkenyl ist und die Alkyl- oder Alkenylgruppe mit 1-2 Carboxyl-, 1-3 Hydroxy-, 1-3 Amino-, 1-3 Phenyl- und/oder 1-3 C₁₋₅-Alkylresten substituiert sein kann. Bevorzugte Carbonsäuren sind Fumarsäure, Malonsäure, Apfelsäure, Succinsäure, Milchsäure, Glycolsäure, Maleinsäure, Zitronensäure, Asparginsäure und Mandelsäure.

Der Wirkstoff Vildagliptin kann in Form seiner freien Base oder, falls gewünscht, in Form eines pharmazeutisch verträglichen Salzes hiervon eingesetzt werden. Pharmazeutisch verträgliche Salze des Vildagliptins sind in der WO 2000/034241 offenbart.

Die Wirkstoffmengen in der erfindungsgemäßen pharmazeutischen Zusammensetzung können vom Fachmann in Abhängigkeit von der gewünschten Dosierung frei gewählt werden. Vorzugsweise enthält die pharmazeutische Zusammensetzung 25 bis weniger als 87 Gew.-% Metforminhydrochlorid, 3 bis 20 Gew.-% Sitagliptin oder Vildagliptin oder ein pharmazeutisch verträgliches Salz eines dieser Wirkstoffe, berechnet auf Basis der freien Base des Wirkstoffs, und mehr als 10 bis 30 Gew.-% Schmiermittel, jeweils bezogen auf das Gesamtgewicht der Zusammensetzung. Hierbei ist zu beachten, dass sich die Gew.-%-Angaben vorliegend, soweit sie sich auf das Gesamtgewicht der Zusammensetzung beziehen, auf das Gewicht der Zusammensetzung beziehen, jedoch ohne gegebenenfalls vorhandene Tablettenüberzüge in Form von Lackschichten, etc.

Bevorzugt werden die Wirkstoffmengen so gewählt, dass eine Dosiseinheit der pharmazeutischen Zusammensetzung 50 mg oder 100 mg Sitagliptin oder Vildagliptin enthält, jeweils berechnet auf Basis der freien Base des Wirkstoffs, sowie 500 mg, 850 mg oder 1000 mg Metforminhydrochlorid. Eine besonders bevorzugte Tablette enthält 50 mg Sitagliptin oder Vildagliptin, berechnet auf Basis der freien Base des Wirkstoff, und 1000 mg Metforminhydrochlorid.

Die erfindungsgemäße pharmazeutische Zusammensetzung enthält außer den Wirkstoffen als zwingenden weiteren Bestandteil mehr als 10 Gew.-% Schmiermittel bezogen auf das Gesamtgewicht der Zusammensetzung. Das Schmiermittel ist dabei entweder Polyethylenglykol oder eine Mischung aus Polyethylenglykol mit einem oder mehreren anderen Schmiermitteln. Vorzugsweise enthält die erfindungsgemäße pharmazeutische Zusammensetzung 12 bis 28 Gew.-%, bevorzugter mehr als 15 bis 28 Gew.-%, beispielsweise 15,1 bis 24 Gew.-%, besonders bevorzugt 16 bis 24 Gew.-%, wie beispielsweise etwa 19 Gew.-% Schmiermittel, jeweils bezogen auf das Gesamtgewicht der Zusammensetzung.

Das eingesetzte Polyethylenglykol hat vorzugsweise ein Molekulargewicht von mindestens 1000 g/mol. Bevorzugt liegt das Molekulargewicht des Polyethylenglykols in einem Bereich von 1000 bis 20000 g/mol, besonders bevorzugt in einem Bereich von 6000 bis 10000 g/mol. Eine bevorzugtes Polyethylenglykol ist PEG 8000.

Wenn das Schmiermittel eine Mischung aus Polyethylenglykol und einem oder mehreren anderen Schmiermitteln ist, so kann das Polyethylenglykol mit herkömmlichen bekannten Schmiermitteln gemischt werden, wie beispielsweise Magnesiumstearat, Calciumstearat, Stearinsäure, Natriumstearylfumarat, hydriertes Rizinusöl, Talkum, Fumarsäure, Stärken, wie beispielsweise Erbsen-, Weizen-, Mais-, Kartoffel-, Roggen-, Reis-, Algen- oder Tapiokastärke, Natriumlaurylsulfat, kolloidales Siliziumdioxid, Magnesiumtrisilicat, Calciumphosphat, Aluminiumstearat, Magnesiumcarbonat, Magnesiumoxid, Cellulose und mikrokristalline Cellulose. Vorzugsweise wird das Polyethylenglykol mit einem oder mehreren anderen Schmiermitteln, ausgewählt aus der Gruppe, bestehend aus Talkum, Stärke und Natriumlaurylsulfat gemischt.

Das Mischungsverhältnis der eingesetzten Schmiermittel kann vom Fachmann in Abhängigkeit von den gewünschten Eigenschaften der pharmazeutischen Zusammensetzung frei gewählt werden. Vorzugsweise sollte die Schmiermittelmischung mindestens 10 Gew.-%, bevorzugt mindestens 50 Gew.-% und besonders bevorzugt mindestens 85 Gew.-% Polyethylenglykol bezogen auf das Gesamtgewicht des Schmiermittels enthalten.

Um die Freisetzungseigenschaften der pharmazeutischen Zusammensetzung zu beeinflussen, kann als Schmiermittelzusatz Natrumlaurylsulfat verwendet werden. In diesem Fall beträgt die Menge des eingesetzten Natriumlaurylsulfats vorzugsweise 0,5 bis 2 Gew.-% bezogen auf das Gesamtgewicht der Zusammensetzung.

Die erfindungsgemäße pharmazeutische Zusammensetzung kann neben den Wirkstoffen und dem Schmiermittel auch weitere übliche pharmazeutische Hilfsstoffe enthalten, wie beispielsweise Antioxidantien, Bindemittel, Emulgatoren, Farbstoffe, Füllmittel und Sprengmittel. In einer bevorzugten Ausführungsform enthält die pharmazeutische Zusammensetzung jedoch außer den beiden Wirkstoffen (wobei der Wirkstoff Metforminhydrochlorid in Mischung mit Aerosil (kolloidale Kieselsäure) vorliegen kann) und dem Schmiermittel keine weiteren Inhaltsstoffe. Besonders bevorzugt besteht die pharmazeutische Zusammensetzung aus den beiden Wirkstoffen, Aerosil und Polyethylenglykol. Alternativ kann die pharmazeutische Zusammensetzung aus den beiden Wirkstoffen, ggf. Aerosil, Polyethylenglycol und einem Bindemittel, beispielsweise Polyvinylpyrrolidon (PVP), bestehen.

Die erfindungsgemäße pharmazeutische Zusammensetzung kann in Form von Tabletten vorliegend. Diese sind vorzugsweise durch Direktverpressen oder Verfahren, enthaltend Nass- oder Schmelzgranulieren erhältlich. Bevorzugt werden die Tabletten durch Direktverpressen erhalten.

Wenn gewünscht, können die Tabletten mit einem oder mehreren Überzügen, beispielsweise einem Filmüberzug versehen sein. Entsprechende Überzüge sind dem Fachmann bekannt.

Schließlich betrifft die vorliegende Erfindung auch ein Verfahren zur Herstellung einer wie vorstehend beschriebenen pharmazeutischen Zusammensetzung, wobei die Wirkstoffe mit dem Schmiermittel und gegebenenfalls weiteren Hilfsstoffen gemischt werden und die so erhaltene Mischung gegebenenfalls nach Sieben und/oder Granulieren zu Tabletten verpresst wird. Vorzugsweise wird die Mischung vor dem Verpressen nicht granuliert, sondern direkt zu Tabletten verpresst. Alternativ kann die Mischung durch Nass- oder Trockengranulation oder Schmelzgranulation zunächst in ein Granulat geformt und anschließend zu Tabletten verpresst werden.

In einer bevorzugten Ausführungsform des erfindungsgemäßen Verfahrens wir der Wassergehalt der Mischung vor dem Verpressen auf 2 bis 3 Gew.-% bezogen auf das Gesamtgewicht der Mischung eingestellt. Hierdurch werden die Eigenschaften der Mischung insbesondere für die Direktverpressung verbessert. Die Einstellung des Wassergehalts kann vor oder nach dem Sieben der Mischung erfolgen.

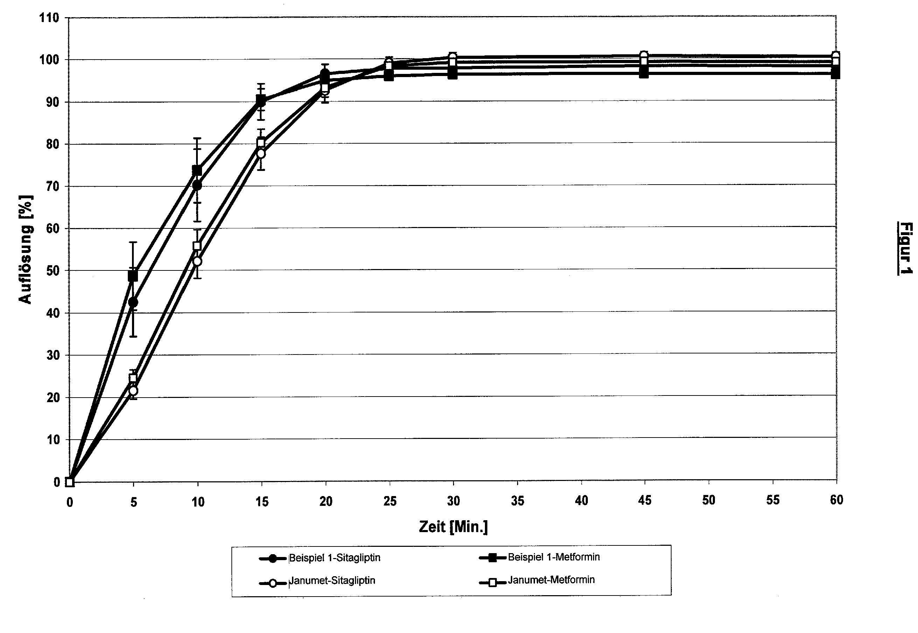

Die anliegende Figur 1 zeigt die Freisetzungsprofile einer erfindungsgemäßen pharmazeutischen Zusammensetzung gemäß Beispiel 1 für die beiden Wirkstoffe Sitagliptin und Metformin im Vergleich zu dem Handelspräparat Janumet^{®}.

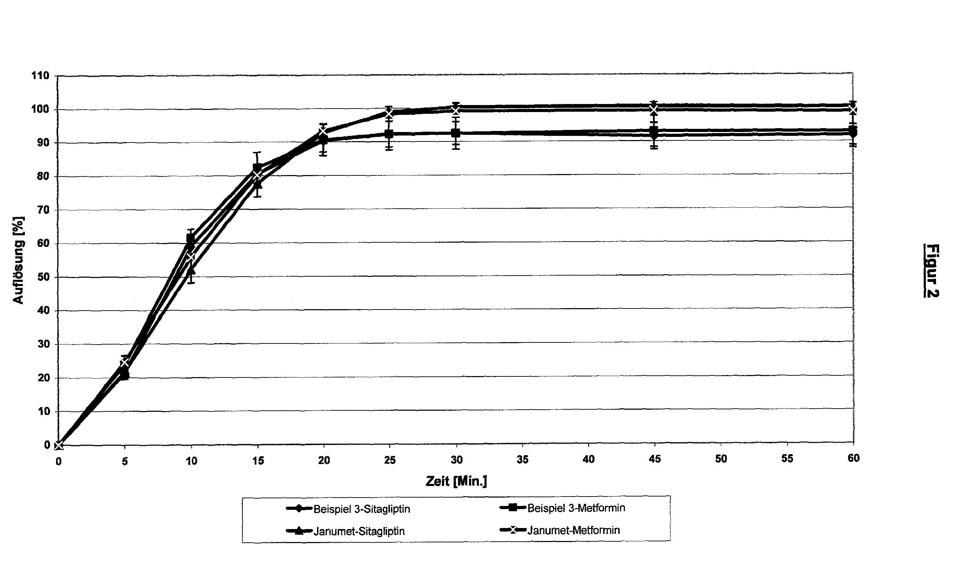

Figur 2 zeigt die Freisetzungsprofile einer erfindungsgemäßen pharmazeutischen Zusammensetzung gemäß Beispiel 3 für die beiden Wirkstoffe Sitagliptin und Metformin im Vergleich zu dem Handelspräparat Janumet^{®}.

Die Erfindung wird nun durch die nachfolgenden Beispiele näher erläutert, ohne dass diese einschränkend ausgelegt werden sollen.

### Beispiel 1

| **Wirk- und Hilfsstoffe** | **[mg/Tablette]** | **[%]** | **Einwaage [g/Ansatz]** |
|---|---|---|---|
| Sitagliptinphosphatmonohydrat (79,2%) | 63,13 | 4,79 | 1,26 |
| Metforminhydrochlorid (99,6%) Aerosil 0,5% | 1004,02 | 76,23 | 20,08 |
| PEG 8000 | 250,00 | 18,98 | 5,00 |

Das Metforminhydrochlorid als Mischung mit 0,5% Aerosil wurde mit Sitagliptinphosphatmonohydrat und PEG 15 Minuten im Freifallmischer bei 23 UpM im Turbula T10B gemischt. Das Gemisch wurde über ein 0,6 mm Sieb gegeben und anschließend auf einer Excenterpresse verpresst. Die Tablettengröße betrug 21x11 mm.

Die Tabletten wurden anschließend in einem Trommelcoater (Lödige LHC 25) mit 0,35 Gew.-% Opadry II (15 Gew.-% in Wasser) beschichtet.

Das Auflösungsprofil der erhaltenen Tabletten wurde für die Wirkstoffe Sitagliptin und Metformin unter Verwendung von 900 ml Phosphatpuffer, pH 6, bei 37°C und 75 UpM nach der Paddlemethode (USP App. II) gemessen. Die Auflösungsprofile für die beiden Wirkstoffe sind in Figur 1 dargestellt, wobei die Auflösungsprofile für die beiden Wirkstoffe Sitagliptin und Metformin für das Handelsprodukt Janumet^{®} zum Vergleich mit dargestellt sind. Man erkennt, dass die erfindungsgemäßen Tabletten die Wirkstoffe sogar schneller freisetzen, als das Handelsprodukt.

### Beispiel 2

| **Wirk- und Hilfsstoffe** | **[mg/Tablette]** | **[%]** | **Einwaage [g/ansatz]** |
|---|---|---|---|
| Vildagliptin | 50,45 | 3,87 | 1,01 |
| Metforminhydrochlorid (99,6%) Aerosil 0,5% | 1004,02 | 76,97 | 20,08 |
| PEG 8000 | 250,00 | 19,16 | 5,00 |

Die Herstellung der Tabletten erfolgte wie in Beispiel 1.

### Beispiel 3

| **Wirk- und Hilfsstoffe** | **[mg/Tablette]** | **[%]** | **Einwaage [g/Ansatz]** |
|---|---|---|---|
| Sitagliptinphosphatmonohydrat (79,2%) | 63,13 | 4,79 | 9,47 |
| Metforminhydrochlorid (99,6%) Aerosil 0,5% | 1004,02 | 76,23 | 150,60 |
| PEG 8000 | 250,00 | 15,18 | 30,00 |
| PVP | 50,00 | 3,80 | 7,50 |

Die Mischung aus Wirk- und Hilfsstoffen wurde aufgeschmolzen und zu einem Granulat verarbeitet. Das Granulat wurde wie in Beispiel 1 zu Tabletten verpresst.

Die Tabletten wurden anschließend in einem Trommelcoater (Lödige LHC 25) mit 0,35 Gew.-% Opadry II (15 Gew.-% in Wasser) beschichtet.

Die Auflösungsprofile der erhaltenen Tabletten wurden wie in Beispiel 1 bestimmt und sind in Figur 2 wiedergegeben.

## Patentansprüche

1. Pharmazeutische Zusammensetzung, umfassend den Wirkstoff Metformin oder ein pharmazeutisch verträgliches Salz davon in Kombination mit einem der Wirkstoffe Sitagliptin oder Vildagliptin oder einem pharmazeutisch verträglichen Salz eines dieser Wirkstoffe sowie mehr als 10 Gew.-% Schmiermittel bezogen auf das Gesamtgewicht der Zusammensetzung, wobei das Schmiermittel Polyethylenglykol oder eine Mischung aus Polyethylenglykol mit einem oder mehreren anderen Schmiermitteln ist.

2. Pharmazeutische Zusammensetzung gemäß Anspruch 1, umfassend den Wirkstoff Metformin als Hydrochloridsalz.

3. Pharmazeutische Zusammensetzung gemäß einem der vorhergehenden Ansprüche, umfassend den Wirkstoff Sitagliptin als Phosphatsalz, insbesondere als Phosphatmonohydrat.

4. Pharmazeutische Zusammensetzung gemäß einem der vorhergehenden Ansprüche, umfassend 25 bis weniger als 87 Gew.-% Metforminhydrochlorid, 3 bis 20 Gew.-% Sitagliptin oder Vildagliptin oder ein pharmazeutisch verträgliches Salz eines dieser Wirkstoffe, berechnet auf Basis der freien Base des Wirkstoffs, und mehr als 10 bis 30 Gew.-% Schmiermittel, jeweils bezogen auf das Gesamtgewicht der Zusammensetzung.

5. Pharmazeutische Zusammensetzung gemäß Anspruch 4, umfassend 12 bis 28 Gew.-%, vorzugsweise mehr als 15 bis 28 Gew.-% Schmiermittel, bezogen auf das Gesamtgewicht der Zusammensetzung.

6. Pharmazeutische Zusammensetzung gemäß einem der vorhergehenden Ansprüche, worin das Schmiermittel Polyethylenglykol mit einem Molekulargewicht von mindestens 1000 g/mol umfasst.

7. Pharmazeutische Zusammensetzung gemäß Anspruch 6, wobei das Schmiermittel Polyethylenglykol mit einem Molekulargewicht von 1000 bis 20000 g/mol, vorzugsweise 6000 bis 10000 g/mol umfasst.

8. Pharmazeutische Zusammensetzung gemäß einem der vorhergehenden Ansprüche, wobei das Schmiermittel eine Mischung aus Polyethylenglykol mit einem oder mehreren anderen Schmiermitteln, ausgewählt aus der Gruppe, bestehend aus Talkum, Stärke und Natriumlaurylsulfat ist.

9. Pharmazeutische Zusammensetzung gemäß Anspruch 8, wobei die Mischung mindestens 10 Gew.-%, bevorzugt mindestens 50 Gew.-% Polyethylenglykol bezogen auf das Gesamtgewicht des Schmiermittels enthält.

10. Pharmazeutische Zusammensetzung gemäß Anspruch 8 oder 9, die 0,5 bis 2 Gew.-% Natriumlaurylsulfat bezogen auf das Gesamtgewicht der Zusammensetzung enthält.

11. Pharmazeutische Zusammensetzung gemäß einem der vorhergehenden Ansprüche, die als Tablette, vorzugsweise als eine durch Direktverpressen oder Nassgranulieren erhältliche Tablette vorliegt.

12. Verfahren zur Herstellung einer pharmazeutischen Zusammensetzung gemäß einem der Ansprüche 1 bis 11, worin die Wirkstoffe mit dem Schmiermittel und gegebenenfalls weiteren Hilfsstoffen gemischt werden und die so erhaltene Mischung gegebenenfalls nach Sieben und/oder Granulieren zu Tabletten verpresst wird.

13. Verfahren nach Anspruch 12, worin der Wassergehalt in der Mischung auf 2 bis 3 Gew.-% bezogen auf das Gesamtgewicht der Mischung eingestellt wird.
